# EUROPÄISCHE PATENTSCHRIFT

(11) **EP 2 196 239 B1**
(45) Veröffentlichungstag und Bekanntmachung des Hinweises auf die Patenterteilung: **12.09.2012**
(21) Anmeldenummer: 09177444.8
(22) Anmeldetag: 30.11.2009
(51) Int. Cl.: A61N 1/372, H04W 60/00, H04W 52/24, H04W 88/02

(54) **Implantierbares medizinisches Gerät mit Mobilfunk-Modem**
Implantable medical device with mobile wireless modem
Dispositif médical implantable avec modem de radiocommunication mobile

(30) Priorität: 15.12.2008 DE 102008054658
(43) Veröffentlichungstag der Anmeldung: 16.06.2010
(73) Patentinhaber: Biotronik CRM Patent AG, 6341 Baar (CH)
(72) Erfinder: Dörr, Thomas, 12437, Berlin (DE)
(74) Vertreter: Lindner-Vogt, Karin L.

(56) Entgegenhaltungen:
- WO-A2-03/095024
- US-A1- 2004 122 489
- US-B1- 6 718 171

## Beschreibung

Die Erfindung betrifft ein dauerimplantierbares elektronisches Implantat, welches dazu ausgebildet ist mindestens einen technischen oder physiologischen Parameter zu erfassen. Insbesondere betrifft die Erfindung implantierbare Impulsgeneratoren wie Herzschrittmacher, Kardioverter/Defibrillatoren oder dergleichen.

Die Fernüberwachung elektronischer Implantate wie Herzschrittmacher, implantierbare Defibrillatoren oder Neurostimulatoren gewinnt zunehmend an Bedeutung. Diese Implantate übertragen dabei in regelmäßigen Abständen (z. B. einmal täglich) oder ereignisgetriggert Daten an ein Fernüberwachungsserver, auf den wiederum über eine Datenverbindung der Arzt zugreifen und so die Fernüberwachung durchführen kann.

Alle derzeit bekannten Lösungen sind dabei auf eine Relaisstation (z. B. BIOTRONIK Cardio Messenger) zur Übertragung der Daten aus dem elektronischen Implantat an ein Datenübertragungsnetzwerk (Mobilfunknetz, Telefonnetz, etc.) angewiesen. Bevorzugt kommt hier eine Übertragung der Daten des elektronischen Implantates zur Relaisstation via MICS-Band (z. T. auch ISM-Band) zum Einsatz.

Nachteil dieser Lösung ist es, dass der Patient ein zusätzliches, kostenintensives externes Zusatzgerät in Reichweite des Implantates mitführen muss, um so die Datenübertragung vom Implantat zum Übertragungsnetzwerk zu gewährleisten. Diese externen Zusatzgeräte sind auch deswegen als Schwachpunkt dieser Übertragungskette anzusehen, da diese häufig vom Patienten nicht korrekt bedient werden oder sich nicht in Reichweite des Implantates befinden (Patientencompliance). Z. T. stoßen diese Geräte auch auf Ablehnung durch den Patienten und haben psychologische Nebenwirkungen, da der Patient ständig an das elektronische Implantat erinnert wird (Angst, Kontrollzwang, etc.).

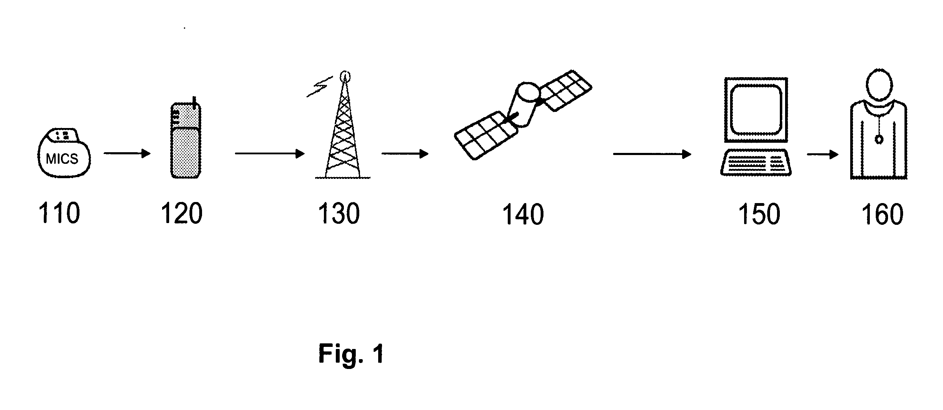

Figur 1 zeigt den derzeitigen Stand der Technik. Ein elektronisches Implantat 110 besitzt einen MICS-Band Transceiver und sendet in periodische Abständen Signale, die Werte technischer und/oder physiologischer Parameter repräsentieren und die mit dem Implantat gemessen wurden, an eine Relaisstation 120, die sich in unmittelbarer Nähe des Patienten mit dem elektronische Implantat zum Übertragungszeitpunkt befinden muss. Diese Relaisstation 120 überträgt dann mittels eines integrierten GSM-Modems die gesendeten Daten an eine GSM-Basisstation 130, die dann wiederum diese Daten über ein Mobilfunknetzwerk 140 an einen Fernüberwachungsserver 150 überträgt und damit dem Arzt 160 zu Verfügung stellt.

Die Relaisstation 120 - genannt Patientengerät - kann in einer mobilen oder stationären Ausführung realisiert sein. Alternativ wird bei einem stationären Patientengerät auch eine Festnetztelefonverbindung für die Datenübertragung verwendet.

Eine sichere Datenübertragung ist nur dann möglich, wenn das Patientengerät 120 eingeschaltet ist und sind in maximal 2-5 Metern Abstand zum elektronischen Implantat befindet.

Der Erfindung liegt die Aufgabe zugrunde, eine Datenverbindung für die Fernüberwachung eines elektronischen Implantates zwischen Implantat und einem Mobilfunknetzwerk (z. B. einem GSM-Netz) ohne eine zusätzliche Relaisstation zu realisieren.

Erfindungsgemäß wird diese Aufgabe gelöst durch ein dauerimplantierbares elektronisches Implantat, welches dazu ausgebildet ist, mindestens einen technischen oder physiologischen Parameter zu erfassen, und welches eine sich erschöpfende Strom- oder Spannungsquelle, also in der Regel eine Batterie, aufweist. Das elektronische Implantat besitzt ein stromarmes Mobilfunk-Modem mit geringer maximaler Sendeleistung, welches mit einer in das elektronische Implantat integrierten Mobilfunk Antenne verbunden ist. Die Angaben "stromarm" und "geringe maximale Sendeleistung" dienen dem besseren Verständnis des Zusammenhangs und beziehen sich auf heute übliche GSM-Modems zur Datenübertragung.
Außerdem besitzt das Implantat eine mit dieser Antenne oder einer zweiten Antenne verbundene stromarme Mobilfunk-Feldstärkemesseinheit und eine Steuereinheit, die mit der Feldstärkemesseinheit und dem Mobilfunk-Modem verbunden ist. Die Steuereinheit ist ausgebildet, eine Einwahl in ein Mobilfunknetz in Abhängigkeit
- sowohl unter Berücksichtigung eines aktuellen Ausgangswertes der Feldstärkemesseinheit (also einem aktuellen Feldstärkewert)
- als auch der Dringlichkeit eines zu übertragenden Dateninhalts nur dann auszulösen, wenn der aktuelle Ausgangswert der Feldstärkemesseinheit einen für eine jeweilige Dringlichkeit spezifische Übertragungsmindesterfolgsquote indiziert.

In einem einfachen Fall kann dies beispielsweise bedeuten, dass die Steuereinheit eine Einwahl nur dann auslöst, wenn die unter Berücksichtigung eines von der Feldstärkemesseinheit bestimmten Mobilfunk-Feldstärkewertes voraussichtlich erforderlichen Sendeleistung einen für eine jeweilige Dringlichkeit spezifischen Höchstwert nicht überschreitet.

Hierbei wird die Integration eines GSM-Modems in ein elektronische Implantat als bekannt vorausgesetzt (siehe: US 2007/0032832 A1 sowie US 2004/0122 489 A1), wobei die aus US 2007/0032832 A1 be kannte Lösung der Erschöpfbarkeit der Batterie eines elektronischen Dauerimplantats keine Rechnung trägt. Demgegenüber erlaubt es die erfindungsgemäße Lösung ein Mobilfunk-Modem in einem elektronischen Implantat so stromarm zu betreiben, das dessen Einsatz gemessen an der zu erwartenden Betriebszeitverkürzung durch die Datenübertragung gerechtfertigt ist.

Die Erfindung beruht auf der Annahme, dass der Träger eines elektronischen Implantates sich in Zukunft immer im Bereich von Mobilfunkzellen, beispielsweise GSM-Zellen aufhält, wobei ein oder mehrfachtäglich auch überdurchschnittlich gute Sende- und Empfangsbedingungen bestehen. Diese sollen mittels der Erfindung ausgenutzt werden und nur bei diesen Bedingungen soll die Verbindung zu einer Mobilfunk-Basisstation überhaupt aufgebaut werden.

Für Patienten, bei denen diese Annahme nicht zutrifft, werden in Zukunft vorrausichtlich sog. Micro-GSM-Zellen für den Betrieb im häuslichen Umfeld von den GSM-Betreibern angeboten.

Vorzugsweise ist das Mobilfunk-Modem ein GSM-Modem und die Mobilfunk-Feldstärkemesseinheit eine GSM-Feldstärkemesseinheit. Das elektronische Implantat enthält vorzugsweise ein Subscriber Identity Modul, beispielsweise in Form einer SIM Karte.

Ferner weist das elektronische Implantat eine Statistik-Einheit auf, die mit der Steuereinheit verbunden oder Teil derselben ist und die ausgebildet ist, eine Einwahlerfolgsstatistik ausgelöster Einwahlen in Abhängigkeit von eines jeweiligen vor einem jeweiligen Einwahlversuch seitens der Mobilfunk-Feldstärkemesseinheit gemessenen Feldstärkewertes aufzuzeichnen. Hierbei kann die Steuereinheit ausgebildet sein, eine Einwahl zusätzlich unter Berücksichtigung der Einwahlerfolgsstatistik für einen jeweils gemessenen Mobilfunk-Feldstärkewert auszulösen.

Weiter ist die Statistikeinheit ausgebildet, die Einwahlerfolgsstatistik unter Berücksichtigung des jeweiligen Einwahlzeitpunktes aufzuzeichnen und die Steuereinheit ist ausgebildet, anhand der Einwahlerfolgsstatistik den Zeitpunkt eines nächsten Einwahlzeitfensters und/oder Feldstärkemessfensters zu bestimmen. So kann die Häufigkeit erfolgreichen Verbindungsaufbaus bei wiederkehrenden Umgebungsbedingungen (z. B. guter GSM-Empfang am Arbeitsplatz) gesteigert werden. Auch kann der Energieaufwand für die Bestimmung der Übertragungsbedingungen durch eine jeweilige Feldstärkemessung reduziert werden.

Die Erfindung ist durch Anspruch 1 definiert.

Gemäß weiterer bevorzugter Ausführungsvarianten weist das elektronische Implantat zwei Strom- oder Spannungsquellen auf, von denen eine für die Versorgung einer Mobilfunk-Einheit unabhängig von übrigen Einheiten des elektronischen Implantates bestimmt ist und die als (vorzugsweise transkutan und drahtlos) wiederaufladbare Batterie ausgebildet ist. Die Mobilfunk-Einheit umfasst in dieser Ausführungsvariante vorzugsweise wenigstens das Mobilfunk-Modem und kann beispielsweise zusätzlich die Mobilfunk-Feldstärkemesseinheit umfassen.

Vorzugsweise besitzt das elektronische Implantat neben der Mobilfunk-Feldstärkemesseinheit einen sehr unspezifischen GSM-Feldstärke-Detektor, der direkt oder vermittels der Steuereinheit dazu ausgebildet ist, die Mobilfunk-Feldstärkemesseinheit nur dann zu aktivieren, wenn der sehr unspezifische GSM-Feldstärke-Detektor eine Mindestfeldstärke (Schwellwert) anzeigt. Das elektronische Implantat kann hierzu einen Verstärker, einen Komparator zum Schwellwertvergleich und Triggereinheit zum Aktivieren der Feldstärkemesseinheit bei Überschreiten des Schwellwertes aufweisen.

Die Steuereinheit ist vorzugsweise dazu ausgebildet, maximal einen Einwahlversuch innerhalb eines einstellbaren Zeitraums, z. B. eines Tages, auszulösen.

Außerdem kann die Steuereinheit dazu ausgebildet sein, die Anzahl der Einwahlversuche innerhalb eines jeweiligen Zeitraums derart zu begrenzen, dass nur ein vorgegebener maximaler Anteil (z. B. 25%) der Kapazität der erschöpfbaren Strom- oder Spannungsquelle für eine Übertragung von Daten mittels des Mobilfunk-Modems verbraucht wird. Hierbei kann die Steuereinheit ausgebildet sein, eine Einwahl im Falle von Notfallnachrichten enthaltenden Dateninhalten unabhängig von der Kapazität der erschöpfbaren Strom- oder Spannungsquelle auszulösen.

Weiterhin kann die Steuereinheit ausgebildet sein, eine Einwahl in ein Mobilfunk-Netz nur für Dateninhalte auszulösen, die durch ein seitens des elektronischen Implantats detektiertes technisches oder physiologisches Ereignis generiert sind. Dies können z. B. Notfallnachrichten sein, die durch die Detektion als gefährlich zu betrachtender Gesundheitszustände ausgelöst sind.

Die Erfindung soll nun anhand eines Ausführungsbeispiels mit Bezug auf die Figuren näher erläutert werden. Von diesen zeigt:
- Fig. 1:: den Stand der Technik;
- Fig. 2:: die erfindungsgemäße Lösung als Gesamtsystem;
- Fig. 3:: ein Blockschaltbild eines elektronischen Implantates mit integriertem GSM-Modem;
- Fig. 4:: einen zweistufigen GSM-Feldstärkedetektor;
- Fig. 5:: das Blockschaltbild einer alternativen Implementierung; und
- Fig. 6:: die in der Beschreibung zu Figur 3 erwähnte Sendeerfolgsstatistik.

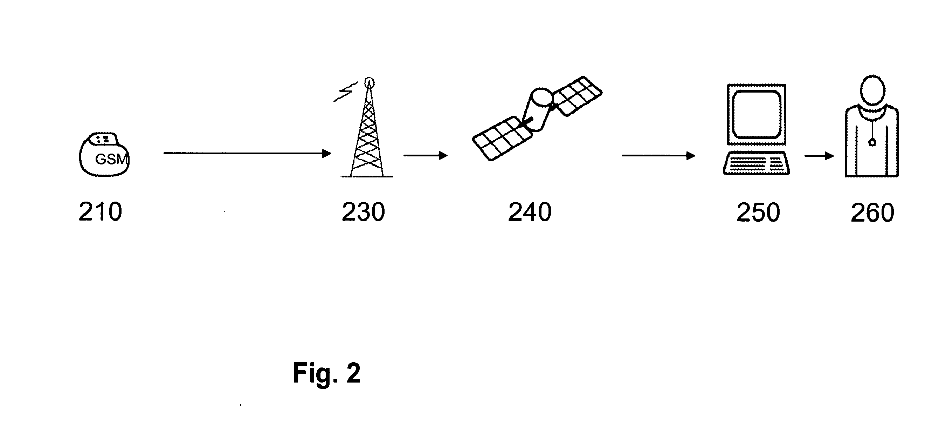

In Figur 2 ist die erfindungsgemäße Lösung als Teil eines Gesamtsystems dargestellt. Dieses umfasst ein elektronisches Implantat 210, in das ein GSM-Modem integriert ist, dass sich gesteuert durch eine Steuereinheit immer dann in eine GSM-Basisstation 230 einwählt, wenn die GSM-Band-Feldstärke eine stromarme Einwahl und Datenübertragung wahrscheinlich macht. Die weitere Übertragungskette mit Mobilfunknetzwerk 240, Fernüberwachungsserver 250 bis zum Arzt 260 bleibt von der Erfindung unbeeinflusst.

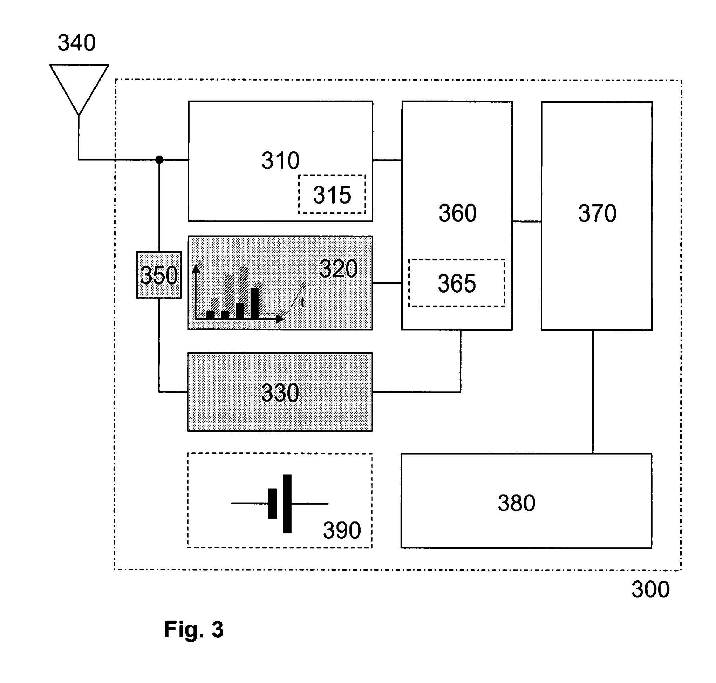

In Figur 3 ist ein Blockschaltbild eines elektronischen Implantates mit integriertem GSM-Modem dargestellt. In ein hermetisch verschlossenes Gehäuse 300 des elektronischen Implantates ist ein stromarmes GSM-Modem 310 inklusive eines SIM-Moduls 315 integriert. Eine Steuereinheit 360 steuert das GSM-Modem 310. Das GSM-Modem 310 ist mit einer GSM-Antenne 340 verbunden. An die GSM-Antenne 340 ist über ein Anpassungsnetzwerk 350 ein extrem stromarmer GSM-Feldstärkedetektor 330 als Feldstärkemesseinheit mit programmierbarer Auslöse-Schwelle angeschlossen. Dieser GSM-Feldstärkedetektor ist ebenfalls mit der Steuereinheit 360 verbunden. Die Steuereinheit ist ihrerseits wiederum ist mit einem Message-Scheduler 370 verbunden.

Ferner enthält das elektronische Implantat eine Diagnostik und Therapiesteuereinheit 380. Diese Diagnostik und Therapiesteuereinheit 380 ist mit dem Message-Scheduler verbunden 370, so dass ausgelöst durch diagnostische oder therapeutische Ereignisse der Message-Scheduler 370 beeinflusst werden kann. Dieser wiederum ist ausgelegt, sowohl ereignisgetriggerte als auch periodische Nachrichtenübertragungen anzufordern. Liegt eine solche Nachrichtenanforderung vor, so wird die Steuereinheit 360 aktiviert. Diese aktiviert dann in periodischen Abständen den Feldstärkedetektor 330 um einen Zeitpunkt zu detektieren, wenn eine GSM-Verbindung mit niedriger Ausgangsleistung hergestellt werden kann. Meldet der Feldstärkedetektor 330 eine geeignete Ausgangsbedingung (z. B. wenn er eine ausreichende Mobilfunk-Feldstärke misst), so aktiviert die Steuereinheit 360 das GSM-Modem 310 zur Einwahl in die Basisstation mit den Identifikationsdaten des SIM-Moduls 315. Die Ausgangsleistung des GSM-Moduls wird an die Signalbedingungen angepasst und ist gegenüber herkömmlichen GSM-Modems deutlich geringer.

Zusätzlich beinhaltet diese Anordnung eine Statistik-Einheit 320 zur statistischen Erfassung des Übertragungserfolges. Diese Statistikeinheit 320 erfasst dabei immer die Übertragungserfolgsrate bezogen auf die vor dem Verbindungsaufbau gemessene GSM-Feldstärke. Diese statistische Erfassung erfolgt dabei bevorzugt als ein 3-dimensionales Histogramm, wobei die dritte Dimension die Tageszeit darstellt. So ist es möglich, Sendebedingungen zu erfassen, die zu bestimmten Tageszeiten wiederkehren (z. B. im Büro o.ä.). Die zeitliche Erfassung kann alternativ auch auf eine ganze Woche erweitert werden.

Die Steuereinheit 360 wiederum beinhaltet eine Einheit zur Klassifikation der Dringlichkeit der Nachricht. Hier wird z. B. zwischen Routinenachrichten (z. B. Remote Follow-up Daten), Nachrichten mittlerer Dringlichkeit (z. B. VF-Episode mit erfolgreicher Therapie) und Notfallnachrichten (z. B. nicht erfolgreicher Maximalenergieschock im ICD) unterschieden. Abhängig von der in dieser Klassifikationseinheit bestimmten Dringlichkeit wird von Controller eine unterschiedliche Sendeerfolgsrate vor dem Verbindungsaufbau vorausgesetzt.

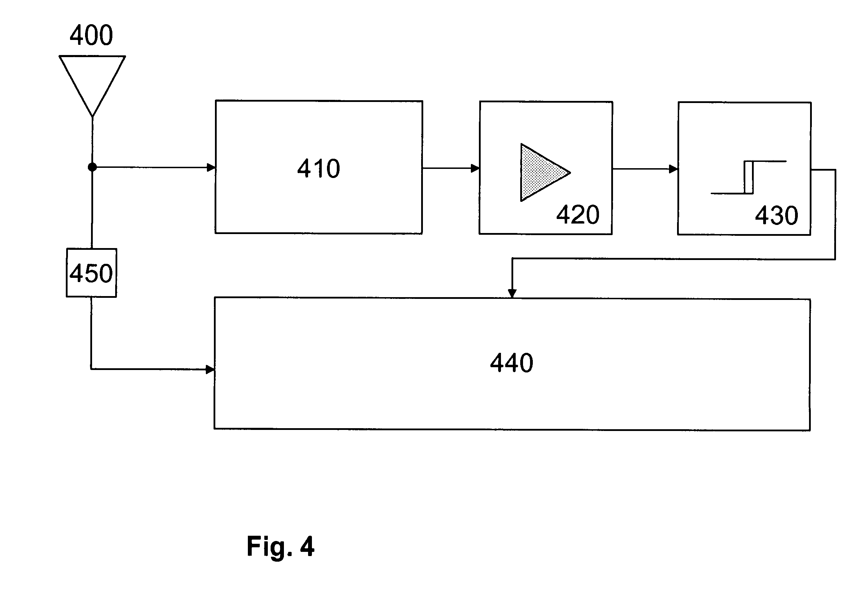

In Figur 4 ist ein zweistufiger GSM-Feldstärkedetektor gemäß einer bevorzugten Ausführungsvariante dargestellt. Ziel ist es, einen extrem stromarmen Detektor zu realisieren. Aus diesem Grund besitzt dieser GSM-Feldstärkedetektor eine erste breitbandige Detektorstufe 410, 420, 430, die als unspezifischer GSM-Feldstärkedetektor dient. Die Antenne 400 ist mit einen breitbandigen Eingangsnetzwerk 410 verbunden. Nach der GSM-Signalverstärkung durch einen Verstärker 420 wird die Signalfeldstärke einer programmierbaren Triggereinheit 430 zur Verfügung gestellt die mittels eines integrierten Komparators einen Schwellwertvergleich durchführt und ggf. ein Aktivierungssignal auslöst. Da Eingangsnetzwerk 410, der Verstärker 420 und die Triggereinheit 430 bilden die erste breitbandige Detektorstufe als unspezifischem GSM-Feldstärkedetektor, der erst wenn das Eingangssignal eine programmierbare Schwelle für eine bestimmte Zeit überschreitet, eine zweite spezifische, frequenzselektive GSM-Feldstärkedetektorstufe aktiviert, die als GSM-Feldstärkemesseinheit 440 dient. Diese ist über ein entsprechend frequenzselektives Netzwerk 450 mit der GSM-Antenne verbunden.

Die frequenzselektive GSM-Feldstärkemesseinheit 440 prüft neben der Feldstärke weitere spezifische Signalcharakteristika z. B. ein jeweiliges Signal-Rausch-Verhältnis, um die Wahrscheinlichkeit einer erfolgreichen GSM-Einwahl zu steigern. Sind diese Prüfungen positive, dann wird ein entsprechendes Ausgangssignal generiert.

Im Folgenden soll dargestellt werden, dass bei Anwendung der erfindungsgemäßen Lösung die Applikation eines GSM-Modems in einem elektronischen Implantat am Beispiel eines implantierbaren Kardioverter/Defibrillators (ICD) möglich ist:

### (A) Stand der Technik:

| | |
|---|---|
| Batteriekapazität: | -2,3 Ah bei einer Betriebsspannung von 3V |
| Typ. Betriebszeit eines ICD: | 6 Jahre |
| Datenübertragungsschema für Fernüberwachung: | 1 Einwahlversuch / Tag + |
| | 5 Ereignisnachrichten / Jahr |

### Strombedarf mit "Standard-GSM-Einwahl (z. B. GPRS)"

| | |
|---|---|
| Einwahldauer: | bis 10sec |
| Stromaufnahme bei einer Betriebsspannung von 3V: | bis 1 A |

Ladung für Fernüberwachung = 370 Einwahlversuche * 6 Jahre * 10sec * 1A = 6,1 Ah
D. h. mit derzeitigem Einwahlprotokoll ist eine GSM-Lösung nicht möglich.

### (B) Erfindungsgemäße Lösung:

| | |
|---|---|
| Batteriekapazität: | ~2,3 Ah bei einer Betriebsspannung von 3V |
| Typ. Betriebszeit eines ICD: | 6 Jahre |
| Datenübertragungsschema für Fernüberwachung: | 1 Einwahlversuch / Tag + |
| | 5 Ereignisnachrichten / Jahr |

### Strombedarf mit "Feldstärkeabhängiger GSM-Einwahl"

| | |
|---|---|
| Einwahldauer: | bis 3 sec |
| Stromaufnahme bei einer Betriebsspannung von 3V: | bis 0,25 A |

Ladung für Fernüberwachung = 370 Einwahlversuche * 6 Jahre * 3sec * 0,25A = 0,5 Ah

Mit der erfindungsgemäßen Lösung ist eine GSM-Einwahl des Implantates möglich. Die Batteriebelastung liegt bei unverändertem Übertragungsschema bei 20% ihrer Kapazität. Diese für die Datenübertragung benötigte Batteriekapazität liegt in der Größenordnung der in den nächsten Jahren zu erwartenden Steigerung der Batteriekapazität bei gleichbleibenden Batterievolumen.

### (C) Erfindungsgemäße Lösung mit reduzierten Datenübertragungsschema:

| | |
|---|---|
| Batteriekapazität: | ~2,3 Ah bei einer Betriebsspannung von 3V |
| Typische Betriebszeit eines ICD: | 6 Jahre |
| Datenübertragungsschema für Fernüberwachung: | 1,5 Einwahlversuche / Woche |
| | 5 Ereignisnachrichten / Jahr |

1,5 Einwahlversuche / Woche bedeutet, es finden max. 2 Einwahlversuche pro Woche statt, ist der erste erfolgreich, dann wird der 2. versuch nicht gestartet. In der Berechnung wird davon ausgegangen, dass der erste Einwahlversuch mit einer 50% Erfolgsrate verbunden ist.

### Strombedarf mit "Feldstärkeabhängiger GSM-Einwahl"

| | |
|---|---|
| Einwahldauer: | bis 3 sec |
| Stromaufnahme bei einer Betriebsspannung von 3V: | bis 0,25 A |

Ladung für Fernüberwachung = 83 Einwahlversuche * 6 Jahre * 3sec * 0,25A = 0,1 Ah

Mit der erfindungsgemäßen Lösung und einem angepassten Datenübertragungsprotokoll ist die Applikation eines GSM-Modems in einen ICD ohne nennenswerte Beschränkung der Betriebsdauer (<5%) des ICD möglich.

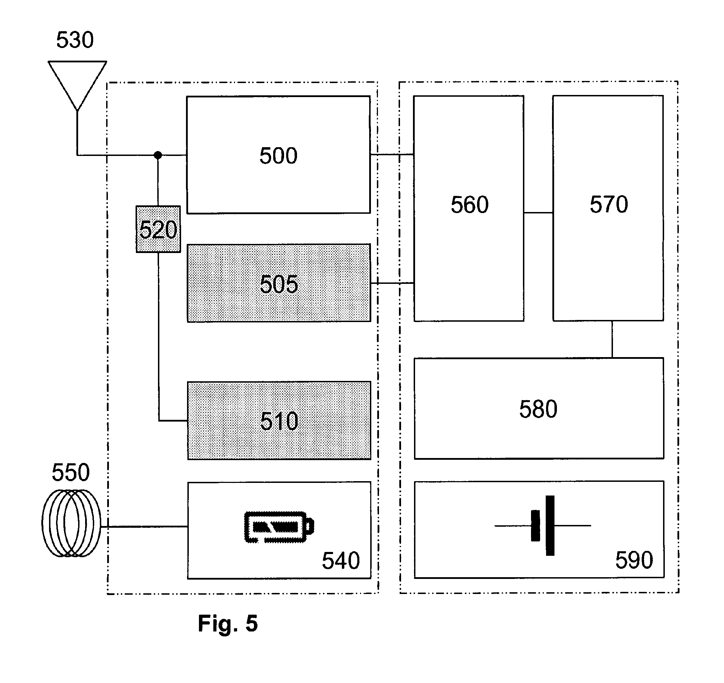

Figur 5 zeigt das Blockschaltbild einer alternativen Implementierung. Gemäß dieser Variante werden das GSM-Modem 500 mit Antenne 530, das SIM-Modul 505 und der Feldstärkedetektor nebst Anpassungsnetzwerk 510, 520 durch eine eigene wiederaufladbare Batterie 540 versorgt. Die Aufladung dieser Batterie 540 erfolgt z. B. über eine außerhalb des metallischen Implantatgehäuses angebrachte Ladespule 550.

Die medizinisch kritischen Funktionen des elektronischen Implantates, d. h. Diagnostik und Therapiesteuereinheit 580 und auch der Message-Scheduler 570 und Controller 560 werden von einer Primärzelle als Strom- oder Spannungsquelle gespeist, so dass die regelmäßig erforderliche Wiederaufladung des Akkus kein sicherheitsrelevantes Problem des Medizinproduktes darstellt, sondern lediglich die Datenübertragung gewährleistet.

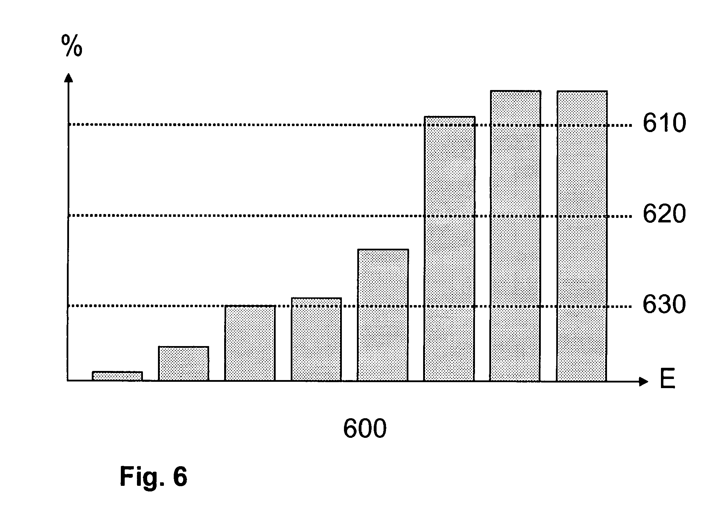

Figur 6 zeigt die in der Beschreibung zu Figur 3 erwähnte Einwahlerfolgsstatistik in Form eines Histogramms 600 von Übertragungserfolgsquoten für eine bestimmte Tageszeit.

In den einzelnen Histogrammklassen wird festgehalten, ob eine Datenübertragung bezogen auf jeweils eine vor dem Verbindungsaufbau gemessenen Feldstärkeklasse erfolgreich beendet werden konnte.

Für die einzelnen Dringlichkeitsklassen der Nachrichten sind unterschiedliche Grenzen (610...630) (Übertragungsmindesterfolgsquoten) für die geforderte Übertragungserfolgsquote festgelegt. Ein Verbindungsaufbau findet nur dann statt, wenn die im Histogramm erfasste Übertragungserfolgsquote die für die jeweilige Dringlichkeit der Nachricht programmierte Schwelle (Übertragungsmindesterfolgsquote) überschreitet. So wird ein Sendeversuch bei einer Notfallnachricht bereits bei einer kleinen Erfolgsquote (630) gestartet, bei einer Routinenachricht hingegen nur bei einer sehr guten Erfolgsquote oberhalb der mit 610 gekennzeichnet Übertragungsmindesterfolgsquote.

Optional kann bei den Notfallnachrichten in Kombination mit einer schlechten Übertragungserfolgsquote (630) die Sendeleistung zusätzlich heraufgesetzt werden.

Die erfindungsgemäße Lösung ist nicht auf die Verwendung von GSM als Übertragungsstandard limitiert, ebenso können weitere Übertragungsverfahren, wie zum Beispiel nach den Mobilfunkstandards UMTS, HSDPA, HSUPA oder LTE, aber auch Bluetooth, WLAN oder ähnliche gleichermaßen an Stelle von GSM verwendet werden.

## Patentansprüche

1. Dauerimplantierbares elektronisches Implantat, welches dazu ausgebildet ist mindestens einen technischen oder physiologischen Parameter zu erfassen, und welches eine sich erschöpfende Strom- oder Spannungsquelle (390) aufweist, sowie:
- ein stromarmes Mobilfunk-Modem (310) mit geringer maximaler Sendeleistung enthält, welches mit einer in das elektronische Implantat integrierten Mobilfunk Antenne (340) verbunden ist,
- einer mit dieser Antenne oder einer zweiten Antenne verbundenen stromarmen Mobilfunk-Feldstärkemesseinheit (330) und
- einer Steuereinheit (360), die mit der Feldstärkemesseinheit 330) und dem Mobilfunk-Modem (310) verbunden und derart ausgebildet ist, eine Einwahl in ein Mobilfunknetz in Abhängigkeit
- sowohl unter Berücksichtigung eines aktuellen Ausgangswertes der Feldstärkemesseinheit (330)
- als auch der Dringlichkeit eines zu übertragenden Dateninhalts nur dann auszulösen, wenn der aktuelle Ausgangswert der Feldstärkemesseinheit (330) eine für eine jeweilige Dringlichkeit spezifische Übertragungsmindesterfolgquote indiziert und
- einer Statistikeinheit (320), die mit der Steuereinheit (360) verbunden oder Teil derselben ist und die ausgebildet ist, eine Einwahlerfolgsstatistik ausgelöster Einwahlen in Abhängigkeit von eines jeweiligen vor einem jeweiligen Einwahlversuch seitens der Mobilfunk-Feldstärkemesseinheit (330) gemessenen Feldstärkewertes aufzuzeichnen;
**dadurch gekennzeichnet,**
**dass** die Statistikeinheit (320) ausgebildet ist, die Einwahlerfolgsstatistik unter Berücksichtigung des jeweiligen Einwahlzeitpunktes aufzuzeichnen und dass die Steuereinheit (360) ausgebildet ist, anhand der Einwahlerfolgsstatistik den Zeitpunkt eines nächsten Einwahlzeitfensters und/oder Feldstärkemessfensters zu bestimmen.

2. Elektronisches Implantat nach Anspruch 1, **dadurch gekennzeichnet, dass** das Mobilfunk-Modem (310) ein GSM-Modem ist und dass die Mobilfunk-Feldstärkemesseinheit (330) eine GSM-Feldstärkemesseinheit ist.

3. Elektronisches Implantat nach Anspruch 1 oder 2, **dadurch gekennzeichnet, dass** das elektronische Implantat ein Subscriber Identity Modul (315) enthält.

4. Elektronisches Implantat nach einem der Ansprüche 1 bis 3, **dadurch gekennzeichnet, dass** die Steuereinheit (360) ausgebildet ist, eine Einwahl zusätzlich unter Berücksichtigung der Einwahlerfolgsstatistik für einen jeweils gemessenen Mobilfunk-Feldstärkewert auszulösen.

5. Elektronisches Implantat nach einem der Ansprüche 1 bis 4, **dadurch gekennzeichnet, dass** das elektronische Implantat zwei Strom- oder Spannungsquellen (540, 590) aufweist, von denen eine (540) für die Versorgung einer Mobilfunk-Einheit unabhängig von übrigen Einheiten des elektronischen Implantates bestimmt ist und die als wiederaufladbare Batterie ausgebildet ist.

6. Elektronisches Implantat nach Anspruch 5, **dadurch gekennzeichnet, dass** die Mobilfunk-Einheit wenigstens das Mobilfunk-Modem (500) umfasst.

7. Elektronisches Implantat nach einem der Ansprüche 1 bis 6, **dadurch gekennzeichnet, dass** das elektronische Implantat neben der Mobilfunk-Feldstärkemesseinheit (440) einen vergleichsweise unspezifischen GSM-Feldstärke-Detektor (410, 430, 430) aufweist, und die Steuereinheit ausgebildet ist, die Mobilfunk-Feldstärkemesseinheit (440) nur dann zu aktivieren, wenn der sehr unspezifische GSM-Feldstärke-Detektor (410, 430, 430) eine Mindestfeldstärke anzeigt.

8. Elektronisches Implantat nach einem der Ansprüche 1 bis 7, **dadurch gekennzeichnet, dass** die Steuereinheit (360) ausgebildet ist, maximal einen Einwahlversuch innerhalb eines einstellbaren Zeitraums auszulösen.

9. Elektronisches Implantat nach einem der Ansprüche 1 bis 8, **dadurch gekennzeichnet, dass** die Steuereinheit (360) ausgebildet ist, die Anzahl der Einwahlversuche innerhalb eines jeweiligen Zeitraums derart zu begrenzen, dass nur ein vorgegebener maximaler Anteil der Kapazität der erschöpfbaren Strom- oder Spannungsquelle (390) für eine Übertragung von Daten mittels des Mobilfunk-Modems verbraucht wird.

10. Elektronisches Implantat nach Anspruch 9, **dadurch gekennzeichnet, dass** die Steuereinheit (360) ausgebildet ist, eine Einwahl im Falle von Notfallnachrichten enthaltenden Dateninhalten unabhängig von der Kapazität der erschöpfbaren Strom- oder Spannungsquelle auszulösen.

11. Elektronisches Implantat nach einem der Ansprüche 1 bis 10, **dadurch gekennzeichnet, dass** die Steuereinheit (360) ausgebildet ist, eine Einwahl in ein Mobilfunk-Netz nur für Dateninhalte auszulösen, die durch ein seitens des elektronischen Implantats detektiertes technisches oder physiologisches Ereignis generiert sind.

## Claims

1. A permanently implantable electronic implant which is configured for detecting at least one technical or physiological parameter and which has an exhaustible current or voltage source (390), and:
- a low-current mobile wireless modem (310) with low maximum transmission power, which is connected to a mobile wireless antenna (340) integrated in the electronic implant,
- a low-current mobile wireless field strength measuring unit (330) connected to said antenna or a second antenna, and
- a control unit (360) which is connected to the field strength measuring unit (330) and the mobile wireless modem (310) and is configured so as to trigger dial-in into a mobile wireless network in dependence on
- an actual output value to be considered of the field strength measuring unit (330)
- as well as the urgency of a data content to be transmitted,
but only if the actual output value of the field strength measuring unit (330) indicates a minimum transmission success rate specific for a respective urgency, and
- a statistic unit (320) which is connected to or is a part of the control unit (360) and which is configured to record a dial-in success statistic of triggered dial-ins in dependence on a respective field strength value measured by the mobile wireless field strength measuring unit (330) prior to a respective dial-in attempt;
**characterized in**
**that** the statistic unit (320) is configured to record the dial-in success statistic in consideration of the respective dial-in time and that the control unit (360) is configured to determine, based on the dial-in success statistic, the time of a next dial-in window and/or field strength measuring window.

2. The electronic implant according to claim 1, **characterized in that** the mobile wireless modem (310) is a GSM modem and that the mobile wireless field strength measuring unit (330) is a GSM field strength measuring unit.

3. The electronic implant according to claim 1 or claim 2, **characterized in that** the electronic implant includes a subscriber identity module (315).

4. The electronic implant according to any one of the claims 1 to 3, **characterized in that** the control unit (360) is configured to trigger dial-in in addition in consideration of the dial-in success statistic for a respective measured mobile wireless field strength value.

5. The electronic implant according to any one of the claims 1 to 4, **characterized in that** the electronic implant has two current or voltage sources (540, 590), one (540) of which is provided for supplying a mobile wireless unit independently of the remaining units of the electronic implant and is configured as a rechargeable battery.

6. The electronic implant according to claim 4, **characterized in that** the mobile wireless unit comprises at least the mobile wireless modem (500).

7. The electronic implant according to any one of the claims 1 to 6, **characterized in that** in addition to the mobile wireless field strength measuring unit (440), the electronic implant has a comparatively unspecific GSM field strength detector (410, 430, 430), and the control unit is configured to activate the mobile wireless field strength measuring unit (440) only if the very unspecific GSM field strength detector (410, 430, 430) indicates a minimum field strength.

8. The electronic implant according to any one of the claims 1 to 7, **characterized in that** the control unit (360) is configured to trigger maximally one dial-in attempt within an adjustable time period.

9. The electronic implant according to any one of the claims 1 to 8, **characterized in that** the control unit (360) is configured to limit the number of dial-in attempts within a respective time period in such a manner that only a predetermined maximum portion of the capacity of the exhaustible current or voltage source (390) is consumed for transmitting data by means of the mobile wireless modem.

10. The electronic implant according to claim 9, **characterized in that** the control unit (360) is configured such that in the case of data contents containing emergency messages, the control unit triggers dial-in independently of the capacity of the exhaustible current or voltage source.

11. The electronic implant according to any one of the claims 1 to 10, **characterized in that** the control unit (360) is configured to trigger dial-in into a mobile wireless network only for data contents which are generated by a technical or physiological event detected by the electronic implant.

## Revendications

1. Implant électronique destiné à être implanté de façon permanente, lequel est configuré pour détecter au moins un paramètre technique ou physiologique et lequel a une source de courant ou de tension épuisable (390), et :
- un modem sans fil mobile à courant faible (310) avec puissance de transmission maximale faible, qui est relié à une antenne sans fil mobile (340) intégrée dans l'implant électronique,
- une unité de mesure d'intensité de champ sans fil mobile à courant faible (330) reliée à ladite antenne ou à une seconde antenne, et
- une unité de commande (360) qui est reliée à l'unité de mesure d'intensité de champ (330) et au modem sans fil mobile (310) et est configurée de façon à déclencher un accès commuté entrant dans un réseau sans fil mobile en fonction de
- une valeur de sortie réelle devant être considérée de l'unité de mesure d'intensité de champ (330)
- ainsi que l'urgence d'un contenu de données devant être transmis,
mais seulement si la valeur de sortie réelle de l'unité de mesure d'intensité de champ (330) indique un taux de succès de transmission minimal spécifique pour une urgence respective, et
- une unité statistique (320) qui est reliée à ou fait partie de l'unité de commande (360) et qui est configurée pour enregistrer une statistique de succès d'accès commuté entrant pour des accès commutés entrant déclenchés en fonction d'une valeur d'intensité de champ respective mesurée par l'unité de mesure d'intensité de champ sans fil mobile (330) avant une tentative d'accès commuté entrant respective ;
**caractérisé par le fait que**
l'unité statistique (320) est configurée pour enregistrer la statistique de succès d'accès commuté entrant en tenant compte du temps d'accès commuté entrant respectif et que l'unité de commande (360) est configurée pour déterminer, sur la base de la statistique de succès d'accès commuté entrant, le temps d'une fenêtre d'accès commuté entrant suivante et/ou d'une fenêtre de mesure d'intensité de champ.

2. Implant électronique selon la revendication 1, **caractérisé par le fait que** le modem sans fil mobile (310) est un modem GSM et que l'unité de mesure d'intensité de champ sans fil mobile (330) est une unité de mesure d'intensité de champ GSM.

3. Implant électronique selon la revendication 1 ou la revendication 2, **caractérisé par le fait que** l'implant électronique comprend un module d'identité d'abonné (315).

4. Implant électronique selon l'une quelconque des revendications 1 à 3, **caractérisé par le fait que** l'unité de commande (360) est configurée pour déclencher un accès commuté entrant en plus en tenant compte de la statistique de succès d'accès commuté entrant pour une valeur d'intensité de champ sans fil mobile mesurée respective.

5. Implant électronique selon l'une quelconque des revendications 1 à 4, **caractérisé par le fait que** l'implant électronique a deux sources de courant ou de tension (540, 590), l'une (540) desquelles est prévue pour alimenter une unité sans fil mobile indépendamment des unités restantes de l'implant électronique et est configurée sous la forme d'une batterie rechargeable.

6. Implant électronique selon la revendication 4, **caractérisé par le fait que** l'unité sans fil mobile comprend au moins le modem sans fil mobile (500).

7. Implant électronique selon l'une quelconque des revendications 1 à 6, **caractérisé par le fait qu'**en plus de l'unité de mesure d'intensité de champ sans fil mobile (440), l'implant électronique a un détecteur d'intensité de champ GSM comparativement imprécis (410, 430, 430), et l'unité de commande est configurée pour activer l'unité de mesure d'intensité de champ sans fil mobile (440) seulement si le détecteur d'intensité de champ GSM très imprécis (410, 430, 430) indique une intensité de champ minimale.

8. Implant électronique selon l'une quelconque des revendications 1 à 7, **caractérisé par le fait que** l'unité de commande (360) est configurée pour déclencher au plus une tentative d'accès commuté entrant au cours d'une période de temps réglable.

9. Implant électronique selon l'une quelconque des revendications 1 à 8, **caractérisé par le fait que** l'unité de commande (360) est configurée pour limiter le nombre de tentatives d'accès commuté entrant au cours d'une période de temps respective, d'une manière telle que seule une partie maximale prédéterminée de la capacité de la source de courant ou de tension épuisable (390) est consommée pour la transmission de données au moyen du modem sans fil mobile.

10. Implant électronique selon la revendication 9, **caractérisé par le fait que** l'unité de commande (360) est configurée de telle sorte que dans le cas de contenus de données contenant des messages d'urgence, l'unité de commande déclenche un accès commuté entrant indépendamment de la capacité de la source de courant ou de tension épuisable.

11. Implant électronique selon l'une quelconque des revendications 1 à 10, **caractérisé par le fait que** l'unité de commande (360) est configurée pour déclencher un accès commuté entrant dans un réseau sans fil mobile seulement pour des contenus de données qui sont générés par un évènement technique ou physiologique détecté par l'implant électronique.
